# EUROPEAN PATENT APPLICATION

(11) **EP 4 459 630 A1**
(43) Date of publication of application: **06.11.2024**
(21) Application number: 23170907.2
(22) Date of filing: 01.05.2023
(51) Int. Cl.: G16H 30/20, G06T 11/00

(54) **MEDICAL IMAGE RECONSTRUCTION**

(71) Applicant: Koninklijke Philips N.V., 5656 AG Eindhoven (NL)
(72) Inventor: SOMMER, Karsten, Eindhoven (NL); KEUPP, Jochen, Eindhoven (NL)
(74) Representative: Philips Intellectual Property & Standards

(57) **Abstract**

Proposed are concepts pertaining to aiding and/or improving the processing of medical images with reconstruction algorithms. In particular, embodiments of the invention may provide a method and/or system which provisions suspensible reconstruction algorithms, that can be restarted and completed at any time in a subsequent workflow, and this may enable a flexible reconstruction scheduling framework to be realized.

## Description

### FIELD OF THE INVENTION

The present invention relates to the field of medical image processing, and in particular to the field of processing medical images with reconstruction algorithms.

### BACKGROUND OF THE INVENTION

Conventional medical imaging reconstruction algorithms require extensive computation times, such as retrospective motion correction or compressed sensing image reconstruction in Magnetic Resonance Imaging (MRI). In some cases, the computation time required to achieve sufficient diagnostic image quality may be difficult to predict at the end of the data acquisition. For example, computation times for retrospective motion correction may vary significantly (depending on the complexity of the patient motion for example).

Long computation times can be problematic in a clinical routine, because they block the reconstruction hardware for subsequent scans in the current subject examination. In some cases, a new scan may not be started when the reconstruction hardware is busy with tasks from previous scans (thus, holding up an entire workflow).

Further, reconstruction processes are typically started immediately during and after data acquisition and thus need to be completed directly after a scan. Consequently, implementation of reconstruction algorithms typically represents a trade-off between achievable image quality and available computation time during and between subsequent scans.

### SUMMARY OF THE INVENTION

The invention is defined by the claims.

According to examples in accordance with an aspect of the invention, there is provided a medical image processing apparatus comprising: an image reconstruction unit configured to process acquired medical imaging data with a plurality of reconstruction algorithms to generate a reconstructed image. The image reconstruction unit is further configured to: execute a first reconstruction algorithm of the plurality of reconstruction algorithms; suspend execution of the first reconstruction algorithm; during suspension of first reconstruction algorithm, execute a second reconstruction algorithm of the plurality of reconstruction algorithms; and subsequent to completion of the second reconstruction algorithm, resume execution of the first reconstruction algorithm.

Proposed concepts thus aim to provide schemes, solutions, concepts, designs, methods and systems pertaining to aiding and/or improving medical image reconstruction. In particular, embodiments of the invention propose the provision of suspensible/suspendible reconstruction algorithms, that can be restarted and completed at any time in a subsequent workflow. By enabling the execution of a second reconstruction algorithm during the suspension of a first reconstruction algorithm, proposed embodiments may support reconstruction job scheduling that aids optimal arrangement of required reconstruction tasks in available time periods.

Purely by way of example, proposed embodiments may enable a flexible reconstruction scheduling framework to be realized by making use of suspensible reconstruction algorithms. At various time points of a medical image reconstruction task (e.g., after a data pre-processing or after a certain number of iterations), an entire state of the algorithm (e.g. parameter value and/or data arrays) may be captured and stored. This may allow the reconstruction task to be resumed at a later point in the clinical workflow (e.g. when sufficient computational resources are available).

In other words, embodiments propose to support the suspension of a first reconstruction algorithm to enable: (i) the execution of a second reconstruction algorithm; and (ii) resumption of the first reconstruction algorithm after completion of the second reconstruction algorithm. This may enable the realisation of a flexible reconstruction scheduling framework to improve medical image reconstruction. For example, embodiments may be used in relation to medical image processing so as to support a medical professional when selecting treatment for a subject. Such embodiments may also support clinical planning. Improved Clinical Decision Support (CDS) may therefore be provided by proposed concepts.

Some embodiments may further comprise a data storage unit. The image reconstruction unit may then be further configured, responsive to suspending execution of the first reconstruction algorithm, store state information in the data storage unit, the state information describing an execution state of the first reconstruction algorithm. In this way, embodiments may be provided with the capability to capture and store the necessary information for suspension (and subsequent resumption) of the first reconstruction algorithm. This may alleviate the requirement to rely on one or more separate data storage systems.

For example, the state information comprises at least one of: a current version of a medical image; one or more algorithm parameter values; one or more algorithm setting values; an iteration identifier; a current version of a data array; and an algorithm state description. Various types and/or forms of data may therefore be captured and stored in order to enable the suspension (and subsequent resumption) of the first reconstruction algorithm.

In some embodiments, the first reconstruction algorithm may comprise an iterative algorithm. The image reconstruction unit may then be configured to suspend execution of the first reconstruction algorithm upon completion of an iteration of the first reconstruction algorithm. Embodiments may therefore be configured to support the suspension (and subsequent resumption) of an iterative reconstruction algorithm.

Some embodiments may further comprise a control unit configured to control the image reconstruction unit to suspend execution of the first reconstruction algorithm according to a control signal supplied from the control unit to the image reconstruction unit. In this way, the suspension of the first reconstruction algorithm may be controllable (via the control signal from the control unit).

By way of example, the control unit may comprise an input interface adapted to receive a control input signal describing at least one of: an execution schedule; and a scheduling limitation for the first reconstruction algorithm, and the control unit may be adapted to generate the control signal for the image reconstruction unit based on the received control input signal. In this way, the control unit may base its control signal on information received via the input interface, wherein the information may relate to an execution schedule and/or a scheduling limitation for the first reconstruction algorithm.

Such embodiments may further comprise a scheduling component configured to analyse use of medical imaging apparatus for acquiring medical imaging data, to determine at least one of: an execution schedule; and a scheduling limitation for the first reconstruction algorithm based the analysis result, and to generate the control input signal for the control unit based on the determined execution schedule and/or scheduling limitation. That is, embodiments may propose a concept of (i) analysing usage to determine the execution schedule and/or scheduling limitation; and (ii) creating the control input signal based on the analysis result(s).

In some embodiments, the input interface may comprise a user interface for receiving the control input signal from a user. Embodiments may thus support a user providing the control input signal for the control unit (e.g. resulting from manual analysis of usage/schedules, etc.)

The medical imaging data may, for example, comprise CT or MRI data.

The apparatus may be remotely located from a user device for assessment of a subject. In this way, a user (such as a medical professional) may have an appropriately arranged system that can receive medical imaging data at a location remotely located from the subject. Embodiments may therefore enable a user to perform medical assessment of a subject using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The apparatus may further include: a server device comprising the image reconstruction unit; and a client device comprising a user-interface. Dedicated data processing means may therefore be employed for the purpose of image reconstruction, thus reducing processing requirements or capabilities of other components or devices of the system.

The apparatus may further include a client device, wherein the client device comprises the image reconstruction unit and a display unit. In other words, a user (such as a doctor or medical professional) may have an appropriately arranged client device (such as a laptop, tablet computer, mobile phone, PDA, etc.) which processes received medical imaging data in order to generate a reconstructed image and generate a display control signal. Purely by way of example, embodiments may therefore provide a medical image processing system that enables imaging of one or more subjects (e.g. patients) from a single location, wherein real-time communication between a subject and user (e.g. nurse or doctor) is provided and can have its functionality extended or modified according to proposed concepts, for example.

It will be understood that processing capabilities may therefore be distributed throughout the system/apparatus in different ways according to predetermined constraints and/or availability of processing resources.

According to another aspect of the invention, there may be provided a a medical image acquisition system comprising: medical imaging apparatus for acquiring medical imaging data; and medical image processing apparatus according to a proposed embodiment.

Proposed embodiments may thus be employed in combination with conventional/existing medical image acquisition and/or processing systems. In this way, embodiments may integrate into legacy systems so as to improve and/or extend their functionality and capabilities. An improved medical image acquisition/processing system may therefore be provided by proposed embodiments.

According to another aspect of the invention, there is provided a method for processing acquired medical imaging data with a plurality of reconstruction algorithms to generate a reconstructed image, the method comprising: executing a first reconstruction algorithm of the plurality of reconstruction algorithms; suspending execution of the first reconstruction algorithm; during suspension of first reconstruction algorithm, executing a second reconstruction algorithm of the plurality of reconstruction algorithms; and subsequent to completion of the second reconstruction algorithm, resuming execution of the first reconstruction algorithm.

According to another aspect, there is provided a computer program product for processing acquired medical imaging data with a plurality of reconstruction algorithms to generate a reconstructed image, wherein the computer program product comprises a computer-readable storage medium having computer-readable program code embodied therewith, the computer-readable program code configured to perform all of the steps of a proposed embodiment.

Thus, there may also be provided a computer system comprising: a computer program product according to proposed embodiment; and one or more processors adapted to perform a method according to a proposed concept by execution of the computer-readable program code of said computer program product.

Thus, there may be proposed concepts for suspensible reconstruction algorithms, that can be restarted and completed at any time in a subsequent workflow. At various time points of a reconstruction task (e.g., after a data pre-processing or after a certain number of iterations), the entire state of the reconstruction algorithm (e.g. key parameters and data arrays) may be saved. This may allow resumption of the task later in a clinical workflow (e.g. when the computational resources are available). By facilitating the use of suspensible reconstruction algorithms, a flexible reconstruction scheduling framework may be realized. The proposed concept(s) may thus enable improved (e.g. more flexible or optimized) medical image reconstruction.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified block diagram of medical image processing apparatus according to an embodiment of the invention;
Fig. 2 depicts an exemplary clinical workflow employing a method for processing acquired medical imaging data with a plurality of reconstruction algorithms according to an embodiment;
Fig. 3 is a simplified flow diagram of a method for processing acquired medical imaging data with a plurality of reconstruction algorithms according to a proposed embodiment; and
Fig. 4 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

The invention proposes concepts for aiding and/or improving the processing of medical images with reconstruction algorithms. In particular, embodiments of the invention may provide a method and/or system which provisions suspensible reconstruction algorithms, that can be restarted and completed at any time in a subsequent workflow, and this may enable a flexible reconstruction scheduling framework to be realized.

In particular, proposed concepts may provide an approach to enabling the execution of a second reconstruction algorithm during the suspension of a first reconstruction algorithm. Accordingly, embodiments may support reconstruction job scheduling which, in turn, enables optimal arrangement of required reconstruction tasks in available time periods.

For instance, a flexible reconstruction scheduling framework may be realized by making use of suspensible reconstruction algorithms: at various time points of a recon job (e.g., after a data pre-processing or after a certain number of iterations), the entire state of the algorithm (key parameters and data arrays) can be saved locally to disk. This allows to resume the job later in the clinical workflow when the computational resources are available. Purely as an example of the proposed concept(s), a suspensible (iterative) retrospective motion correction algorithm has been implemented by saving the current image and motion parameter estimates as well as certain optimizer settings (e.g. step sizes) to a data storage unit at the end of each iteration. If there is an issue with the reconstruction resources, for example when a new scan needs to be started, the current reconstruction can be stopped without losing the information from the calculations so far. In case of such an interruption, the computation can be resumed later restarting at the last completed iteration, i.e., with negligible computational overhead.

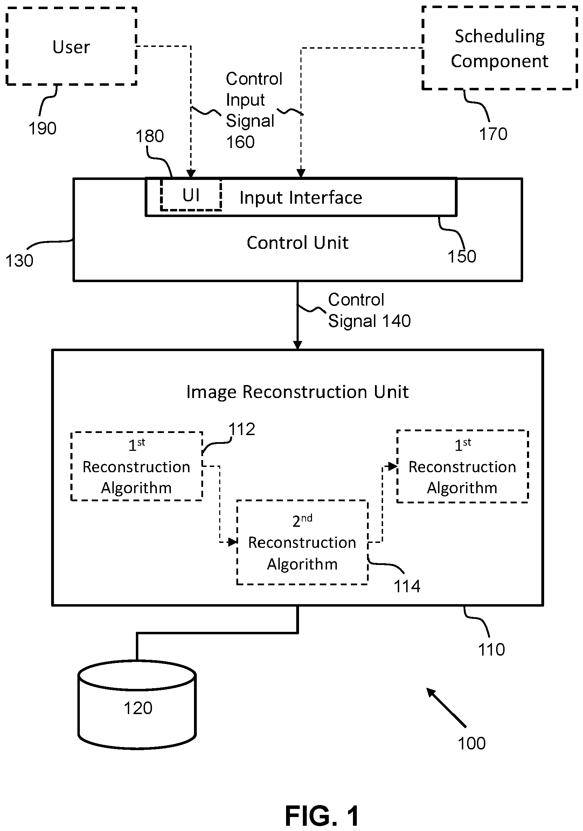

By way of example, a proposed embodiment will now be described with reference to Fig. 1.

Fig. 1 is a simplified block diagram of medical image processing apparatus 100 according to an embodiment of the invention.

The medical image processing apparatus 100 comprises an image reconstruction unit 110 configured to process acquired medical imaging data with a plurality of reconstruction algorithms to generate a reconstructed image. Here, the medical imaging data comprises CT or MRI data (i.e. image data captured using a CT or MRI scanner).

The image reconstruction unit 110 is configured to execute a first reconstruction algorithm 112. The image reconstruction unit 110 is also configured to suspend execution of the first reconstruction algorithm 112, and, during suspension of first reconstruction algorithm 112, to execute a second, different reconstruction algorithm 114. Specifically, in this example, the first reconstruction algorithm 112 comprises an iterative algorithm, and the image reconstruction unit 110 is configured to suspend execution of the first reconstruction algorithm 112 upon completion of an iteration of the first reconstruction algorithm 112.

After completion of the second reconstruction algorithm, the image reconstruction unit 110 resumes execution of the first reconstruction algorithm 112. That is, the image reconstruction unit 110 is configured to facilitate suspensibility of a reconstruction algorithm, thereby enabling another reconstruction algorithm to be executed during the period of suspension of the first reconstruction algorithm.

To support such suspensibility function, the image processing apparatus 100 comprises a data storage unit 120. Responsive to suspending execution of the first reconstruction algorithm 112, the image reconstruction unit stores state information in the data storage unit 120. By way of example, the state information describes an execution state of the first reconstruction algorithm 112, thus providing information/data required to resume the first reconstruction algorithm 112 at a later time. For instance, the state information in this exemplary embodiment comprises at least one of: a current version of a medical image; one or more algorithm parameter values; one or more algorithm setting values; an iteration identifier; a current version of a data array; and an algorithm state description.

In the embodiment of Fig. 1, the medical image processing apparatus 100 also comprises a control unit 130. The control unit 130 is configured to control the image reconstruction unit 110 to suspend execution of the first reconstruction algorithm 112 according to a control signal 140 supplied from the control unit 130 to the image reconstruction unit 110. The medical image processing apparatus 100 is thus provided with a control functionality for controlling the suspension of reconstruction algorithms.

Here, the control unit 130 comprises an input interface 150 that is configured to receive a control input signal 160 describing at least one of: an execution schedule; and a scheduling limitation for the first reconstruction algorithm. The control unit 130 is adapted to generate the control signal 140 for the image reconstruction unit 110 based on the received control input signal. Put another way, the control unit 130 is configured to receive control information (i.e. control input signal 160) that is used to determine the control signal 140 for the image reconstruction unit 110. In this way, a framework for controlling the suspension of reconstruction algorithms may be provided, wherein such control defined according to information provided to the control unit. The information used to define the control may, for example, come from an external system and/or a user, thus provided a wide range of factors and/or aspects that may be used to control suspension of reconstruction algorithms by the image reconstruction unit 110.

By way of demonstrating the potential control options, Fig. 1 also depicts additional features (using dashed lines) that may be included in the medical image processing apparatus 100.

In particular, the embodiment of Fig. 1 may further comprise a scheduling component 170. The scheduling component 170 is configured to analyse use of medical imaging apparatus for acquiring medical imaging data, to determine at least one of: an execution schedule; and a scheduling limitation for the first reconstruction algorithm based the analysis result. Based on the determined execution schedule and/or scheduling limitation, the scheduling component 170 generates the control input signal 160 for control unit 130.

The scheduling component 170 may, for example, automatically analyze the CT/MRI scanner usage at a clinical site (e.g. using log file parsing). In particular, idle times, i.e., extended time periods in which no data acquisition takes place, may be monitored. A learnable algorithm (e.g., a neural network) can then be used to enable prediction of typical idle times, for example during turnover from one patient to the next. This prediction, along with estimated computation times for all recon jobs to be completed, is taken into account by the scheduling component.

Further, for each clinical site, specific requirements about completion of reconstruction tasks may be considered by the scheduling component 170. For example, a clinical workflow may require availability of reconstructed images immediately after the exam (e.g., in emergency departments). At other sites, radiologists may routinely analyze diagnostic images after the last exam of the day, leaving more time for image reconstruction. Apart from these general requirements, individual reconstruction tasks may be prioritized manually by the operator, e.g., if immediate diagnosis or quality control is needed, e.g., while the patient is still on the table. Such a prioritization may also be a default setting of the protocol.

The scheduling component 170 may take both predicted idle times and reconstruction task durations into account, as well as requirements of the reconstruction task(s). In particular, reconstruction algorithm settings, such as total number of iterations, may be automatically adjusted to optimize the image quality for the available computation time. Intermediate (preview) images may be provided at earlier timepoints, if needed, while the final image with optimal quality can be reconstructed a suitable later time point.

Also, the input interface of Fig. 1 may further comprise a user interface for receiving the control input signal from a user. That is, the embodiment may facilitate the provision of a control input signal 160 from a user, thereby enabling human provision of information that may be used to control the image reconstruction unit 110 (via the control unit).

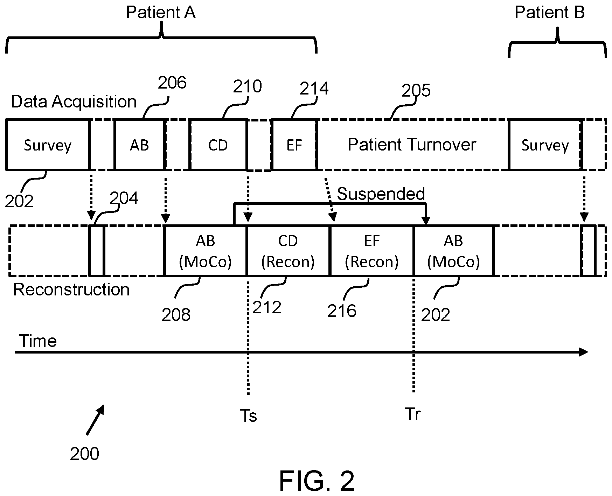

By way of yet further explanation of the proposed concept(s), an exemplary workflow that utilizes the proposed flexible reconstruction framework will now be described with reference to Fig. 2.

Fig. 2 depicts an exemplary clinical workflow employing a method for processing acquired medical imaging data with a plurality of reconstruction algorithms according to an embodiment.

Data acquisition processes are illustrated in the upper/top timeline, and associated image reconstruction process are illustrated in the lower/bottom timeline.

Here, it is assumed that local guidelines require all reconstructions processes/tasks for a specific exam (i.e. Patient A) to be completed before the subsequent exam (i.e. Patient B). In addition, a manual prioritization of a reconstruction process CD(Recon) 212 by the operator is assumed, causing the motion correction process AB(MoCo) 208 to be suspended by the scheduling framework. It is continued during the idle time (i.e. patient turnover period 205) when patient turnover takes place and is completed before the next exam starts. That is, processes are undertaken for a first subject (`Patient A'), followed by a patient turnover period 205. Once the patient turnover period 205 is completed, processes are undertaken for a second subject (`Patient B').

More specifically, for patient A, an initial survey 202 is completed, and an associated reconstruction process 204 (using the results of the survey) is undertaken immediately upon completion of the initial survey 202. Next, a first data acquisition process AB 206 is undertaken. An associated motion correction process AB(MoCo) 208 is undertaken immediately upon completion of the first data acquisition process AB 206.

While the motion correction process AB(MoCo) 208 is executing, a second data acquisition process CD 210 is undertaken. An associated reconstruction process CD(Recon) 212 is undertaken immediately upon completion of the second data acquisition process CD 210. However, so that the associated reconstruction process CD(Recon) 212 can be executed immediately upon completion of the second data acquisition process CD 210, the motion correction process AB(MoCo) 208 is suspended (at Time = Ts).

While the reconstruction process CD(Recon) 212 is executing, a third data acquisition process EF 214 is undertaken. An associated reconstruction process EF(Recon) 216 is undertaken as soon as possible after completion of both the third data acquisition process EF 214 and the reconstruction process CD(Recon) 212. Upon completion of the reconstruction process EF(Recon), the motion correction process AB(MoCo) 208 is resumed (at Time = Ts).

That is, motion correction process AB(MoCo) 208 is suspended automatically due to a (manual) prioritization of the reconstruction process CD(Recon) 212 by the operator. Further, as required by local guidelines, all reconstruction processes are completed at the beginning of the following data acquisition processes for the second (`Patient B').

For the sake of simplicity, parallelized execution of reconstruction tasks is not considered in the example Fig. 2, which can occur, e.g., when only a single GPU is available but is required by all reconstruction algorithms. Nevertheless, extension of the framework to parallelized execution is straightforward. With a parallel reconstruction architecture, there is more flexibility to arrange the reconstruction tasks. However, in practice, the maximum amount of parallel processing may be limited (by the overall compute power) and suspensible reconstruction tasks will allow to better distribute the computation over the available timeslots. Thus, proposed embodiments reduce a need for maximum compute power and thus may be used for cost savings.

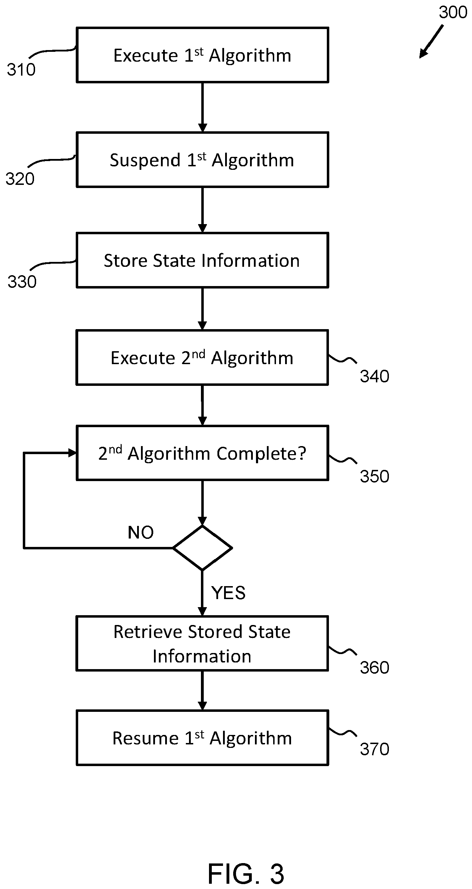

Referring now to Fig. 3, there is depicted a simplified flow diagram of a method for processing acquired medical imaging data with a plurality of reconstruction algorithms according to a proposed embodiment.

The method 300 begins with executing 310 a first reconstruction algorithm of the plurality of reconstruction algorithms.

Next, the method comprises suspending 320 execution of the first reconstruction algorithm. Here, because the first reconstruction algorithm comprises an iterative algorithm, suspending 320 execution of the first reconstruction algorithm is dependent upon completion of an iteration of the first reconstruction algorithm.

Responsive to suspending 320 execution of the first reconstruction algorithm, the method then proceeds to the step of storing 330 state information in a data storage unit, wherein the state information describes an execution state of the first reconstruction algorithm.

Next, during suspension of first reconstruction algorithm, the method proceeds to the step of executing 340 a second reconstruction algorithm of the plurality of reconstruction algorithms. It is then determined, in step 350, whether or not the execution of the second reconstruction algorithm is completed. If it is determined that the execution of the second reconstruction algorithm is not complete, the method returns to step 350 for a subsequent check. If it is determined in step 350 that execution of the second reconstruction algorithm is complete, the method proceeds to step 360. Thus, the method loops until execution of the second reconstruction algorithm is completed.

In step 360 (i.e. subsequent to completion of the second reconstruction algorithm), the stored state information is retrieved from the data storage unit. The, using the retrieved state information, execution of the first reconstruction algorithm is resumed in step 370.

Considering the design and application of the proposed concept(s), the following additional embodiments may be considered:
- If a camera-based workflow support system is available, more accurate predictions about scanner idle times can be made by taking the state of the exam preparation (e.g., patient on table, coil placement completed, table moving, ...) into account.
- Scheduling of reconstruction tasks/processes may be further optimized by receiving information about planned subsequent exams from the radiological information system (RIS). As an example, if the current exam is the last one planned for the day, reconstruction algorithm settings may be selected to maximize image quality regardless of computation time.
- In case of very computationally expensive reconstruction algorithms, suspended reconstruction tasks may be continued on different hardware, such as a dedicated hospital computation cluster, or the completion can be outsourced to a cloud computing environment, to avoid blocking of the scanner's reconstruction resources.

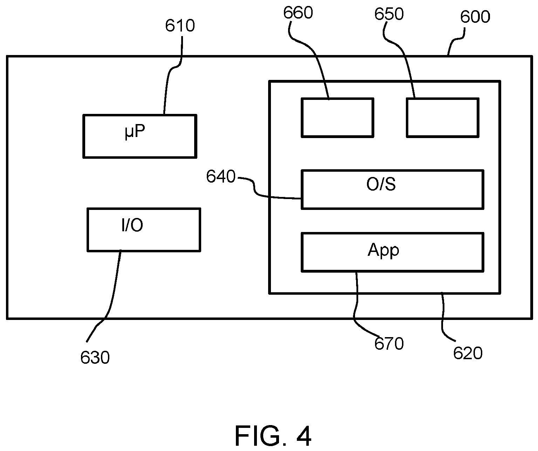

Fig. 4 illustrates an example of a computer 600 within which one or more parts of an embodiment may be employed. Various operations discussed above may utilize the capabilities of the computer 600. For example, one or more parts of the proposed medical image processing apparatus may be incorporated in any element, module, application, and/or component discussed herein. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620, and one or more I/O devices 670 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor-based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 640, source code 630, and one or more applications 660 in accordance with exemplary embodiments. As illustrated, the application 660 comprises numerous functional components for implementing the features and operations of the exemplary embodiments. The application 660 of the computer 600 may represent various applications, computational units, logic, functional units, processes, operations, virtual entities, and/or modules in accordance with exemplary embodiments, but the application 660 is not meant to be a limitation.

The operating system 650 controls the execution of other computer programs, and provides scheduling, input-output control, file and data management, memory management, and communication control and related services. It is contemplated by the inventors that the application 660 for implementing exemplary embodiments may be applicable on all commercially available operating systems.

Application 660 may be a source program, executable program (object code), script, or any other entity comprising a set of instructions to be performed. When a source program, then the program is usually translated via a compiler (such as the compiler 640), assembler, interpreter, or the like, which may or may not be included within the memory 620, so as to operate properly in connection with the O/S 650. Furthermore, the application 660 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 670 may include input devices such as, for example but not limited to, a mouse, keyboard, scanner, microphone, camera, etc. Furthermore, the I/O devices 670 may also include output devices, for example but not limited to a printer, display, etc. Finally, the I/O devices 670 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 670 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at startup, start the O/S 650, and support the transfer of data among the hardware devices. The BIOS is stored in some type of read-onlymemory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 660 and the O/S 650 are read, in whole or in part, by the processor 610, perhaps buffered within the processor 610, and then executed.

When the application 660 is implemented in software it should be noted that the application 660 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 660 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The apparatus of Fig. 1, and the method of Fig. 3, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

Data describing an alertness value determined according to an embodiment may be stored on a storage medium. Task execution data according to an embodiment may be stored on the same storage medium or a different storage medium. Such data may be transmitted as a signal modulated onto an electromagnetic carrier wave. The signal may be defined according to a standard for digital communications. The carrier wave may be an optical carrier, a radio-frequency wave, a millimeter wave, or a near field communications wave. It may be wired or wireless.

To the extent that an embodiment is implemented partly or wholly in hardware, the blocks shown in the block diagram of Fig. 1 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. Medical image processing apparatus (100) comprising:
an image reconstruction unit (110) configured to process acquired medical imaging data with a plurality of reconstruction algorithms to generate a reconstructed image,
wherein the image reconstruction unit (110) is further configured to:
execute (310) a first reconstruction algorithm of the plurality of reconstruction algorithms;
suspend (320) execution of the first reconstruction algorithm;
during suspension of first reconstruction algorithm, execute (340) a second reconstruction algorithm of the plurality of reconstruction algorithms; and
subsequent to completion of the second reconstruction algorithm, resume (370) execution of the first reconstruction algorithm.

2. The medical image processing apparatus of claim 1, further comprising a data storage unit (120),
and wherein the image reconstruction unit (110) is further configured, responsive to suspending execution of the first reconstruction algorithm, store (330) state information in the data storage unit, the state information describing an execution state of the first reconstruction algorithm.

3. The medical image processing apparatus of claim 2, wherein the state information comprises at least one of:
a current version of a medical image;
one or more algorithm parameter values;
one or more algorithm setting values;
an iteration identifier;
a current version of a data array; and
an algorithm state description.

4. The medical image processing apparatus of any of claims 1 to 3, wherein the first reconstruction algorithm comprises an iterative algorithm,
and wherein the image reconstruction unit (110) is configured to suspend execution of the first reconstruction algorithm upon completion of an iteration of the first reconstruction algorithm.

5. The medical image processing apparatus of any of claims 1 to 4, further comprising a control unit (130) configured to control the image reconstruction unit to suspend execution of the first reconstruction algorithm according to a control signal (140) supplied from the control unit to the image reconstruction unit.

6. The medical image processing apparatus of claim 5, wherein the control unit comprises an input interface (150) adapted to receive a control input signal (160) describing at least one of: an execution schedule; and a scheduling limitation for the first reconstruction algorithm,
and wherein the control unit (130) is adapted to generate the control signal for the image reconstruction unit (110) based on the received control input signal.

7. The medical image processing apparatus of claim 6, further comprising:
a scheduling component (170) configured to analyse use of medical imaging apparatus for acquiring medical imaging data, to determine at least one of: an execution schedule; and a scheduling limitation for the first reconstruction algorithm based on the analysis result, and to generate the control input signal (160) for the control unit (130) based on the determined execution schedule and/or scheduling limitation.

8. The medical image processing apparatus of claim 5 or 6, wherein the input interface (150) comprises a user interface (180) for receiving the control input signal from a user (190).

9. The medical image processing apparatus of any of claims 1 to 7, wherein the medical imaging data comprises CT or MRI data.

10. A medical image acquisition system comprising:
medical imaging apparatus for acquiring medical imaging data; and
medical image processing apparatus (100) according to any of claims 1 to 9.

11. A method for processing acquired medical imaging data with a plurality of reconstruction algorithms to generate a reconstructed image, the method comprising:
executing (310) a first reconstruction algorithm of the plurality of reconstruction algorithms;
suspending (320) execution of the first reconstruction algorithm;
during suspension of first reconstruction algorithm, executing (340) a second reconstruction algorithm of the plurality of reconstruction algorithms; and
subsequent to completion of the second reconstruction algorithm, resuming (370) execution of the first reconstruction algorithm.

12. The method of claim 11, further comprising:
responsive to suspending execution of the first reconstruction algorithm, storing (330) state information in a data storage unit, the state information describing an execution state of the first reconstruction algorithm.

13. The method of claim 12, wherein the first reconstruction algorithm comprises an iterative algorithm, and wherein suspending execution of the first reconstruction algorithm is dependent upon completion of an iteration of the first reconstruction algorithm.

14. The method of any of claims 11 to 13, further comprising: controlling the step of suspending execution of the first reconstruction algorithm according to a control signal, and optionally wherein the control input signal is based on at least one of an execution schedule and/or scheduling limitation for the first reconstruction algorithm.

15. A computer program comprising computer program code means adapted, when said computer program is run on a computer, to implement the method of any of claims 11 to 14.
